Europäisches Patentamt

European Patent Office

Office européen des brevets

(19)

(11) Publication number: **0 175 326**
A2

(12) **EUROPEAN PATENT APPLICATION**

(21) Application number: **85111691.3**

(22) Date of filing: **16.09.85**

(51) Int. Cl.⁴: **B 01 L 3/00**, A 61 B 10/00, G 01 N 1/28, G 01 N 33/48

(30) Priority: **17.09.84 US 651380**

(43) Date of publication of application: **26.03.86**
**Bulletin 86/13**

(84) Designated Contracting States: **AT BE CH DE FR GB IT LI NL SE**

(71) Applicant: **Orion Corporation Ltd, Saunatontuntie 1, SF-02100 Espoo (FI)**

(72) Inventor: **Novgrad, Cliff, 320 Brook Drive, Milltown New Jersey 08850 (US)**
Inventor: **Häivä, Antti Veli-Mies, Ristiaallokonkatu 4 F 132, SF-02320 Espoo (FI)**
Inventor: **Niskanen, Aimo Juhani, Peipontie 20, SF-02210 Espoo (FI)**
Inventor: **Walienius, Kaija Orvokki, Kuunsirppi 2 B 53, SF-02210 Espoo (FI)**
Inventor: **Väänänen, Terttu Sinikka, Meteorinkatu 4 B 37, SF-02210 Espoo (FI)**
Inventor: **West, Cheryl Karin, 3 Braintree Street, Flemington, NJ 08824 (US)**

(74) Representative: **Vossius Vossius Tauchner Heunemann Rauh, Siebertstrasse 4 P.O. Box 86 07 67, D-8000 München 86 (DE)**

(54) **Diagnostic-test specimen-preparation vial.**

(57) A specimen-preparation vial for collecting and preparing a specimen of fecal matter or other clinical specimen for a diagnostic test includes a sample-dilution container (20) and a specimen-collection/dispenser top (50). The sample dilution container (20) is shaped to contain a liquid in an interior volume. The container (20) has an opening (34) dimensioned to receive the specimen and a sidewall which is yieldable upon manual squeezing. The specimen-collection/dispenser top (50) includes a cap housing (110) which is connectable to the specimen-dilution container (20) for closing the opening (34) of the container (20). A specimen-collection tool (130) is connected to the cap housing (110) and projects from the cap housing (110) so that the tool (130) extends into the interior volume of the specimen-dilution container (20) when the cap housing (110) closes the opening (34) of the container (20). The specimen-collection/dispenser top (50) also includes a dispenser tip connected to the cap housing (110). The dispenser tip (112) has a channel passing through it which communicates with the interior volume of the sample-dilution container (20) when the cap housing (110) closes the opening (34) of the container (20). An end of the channel defines a filtrate-discharge orifice. A dispenser-tip closure is provided for closing the filtrate-discharge orifice of the channel passing through the dispenser tip (112). A microfiltration filter is located in the cap housing (110) in a liquid path extending from the interior volume of the sample-dilution container (20) to the filtrate-discharge orifice of the dispenser tip (112) so that liquid flowing from the interior volume of the container to the filtrate-discharge orifice passes through the microfiltration filter. The microfiltration filter has pores passing through it of dimensions effective to permit virus particle to pass through the pores and to block the passage of particles significantly larger than virus particles.

Our ref.: T 848 EP
Case: US 651,380
Orion Corporation Ltd.

1 6. SEP. 1985

## DIAGNOSTIC-TEST SPECIMEN-PREPARATION VIAL

### Technical Field

The present invention relates to a vial for use in preparing specimens for diagnostic tests such as medical tests for detecting certain viruses in fecal matter or other clinical specimens.

### Background Art

Gastroentestinal diseases are among the most prevalent types of illness in infants and young children. Only recently have specific virus infections been identified as a major cause of many of these diseases.

One virus, a rotavirus, has been identified as a principal cause of sporadic acute winter gastroenteritis in infants and young children. Children who contract this infection frequently suffer from severe diarrhea and fever, occasionally accompanied by vomiting. In severe cases, the disease can be fatal. It has been estimated that about half of the infants treated for acute winter gastroenteritis are suffering from a gastroenteritis caused by this virus. The disease is a world-wide problem and is a significant cause of childhood deaths in developing countries.

Diagnosis of gastroenteritis caused by a rotaviral infection is based on the presence of rotavirus particles in the feces. Rotavirus particles are generally shed in large quantities during gastroentestinal rotaviral infections. Rotavirus particles are too small to be observed directly with an optical microscope. However, the characteristically wheel-shaped particles can be observed with an electron microscope. In addition, rotavirus particles can be identified by a number of immunochemical diagnostic procedures, including latex-bound-antibody agglutination, immunofluorescence, and enzyme-linked immunosorbent assay techniques.

Typically, diagnostic procedures for detecting the presence of rotavirus particles in fecal matter involve a number of preliminary specimen preparation steps which have heretofore been a source of troublesome problems. Ordinarily a specimen of fecal matter must be collected from a soiled diaper or bedpan of an afflicted person using a disposable spatula and transferred to a specimen-container for transport to a clinical laboratory. In the laboratory the specimen is usually mixed with a buffer solution to disperse the fecal matter and any virus particles which may be present in the solution. The mixing step is often carried out in the specimen container used to transport the specimen to the laboratory or in a laboratory beaker. Many test procedures further require that any gross debris in the solution of fecal matter be removed. Such gross debris is often removed by centrifugation, in which case the solution must be transferred to a centrifuge tube and spun in a centrifuge. The supernatant liquid in the centrifuge tube is then used to prepare slides for examination with an electron microscope or used in an immunochemical diagnostic test procedure.

The conventional specimen-preparation procedure described above has a number of significant drawbacks. The procedure generally involves transferring fecal material or solutions containing fecal material from container to container; which is unpleasant for nursing and laboratory personnel and carries a risk of contamination and infection. Furthermore, the various implements and containers involved must be disposed of or cleaned and sterilized for reuse. The supernatant liquid obtained in the centrifugation step generally includes bacteria or parasitic organisms from the fecal material in addition to whatever virus particles are present. In certain immunochemical diagnostic test procedures for rotaviruses, certain bacteria or other non-viral microorganisms which are occasionally present in fecal matter can interact with the test reagents to give rise to ambiguous or even erroneous test results.

International patent application No. PCT/BR82/00013 published under International Publication No. WO 83/01194 on April 14, 1983 by the World Intellectual Property Organization (the '194 publication) discloses a disposable container for collecting a specimen of fecal matter and preparing it for parasitological examination. The container includes a semiflexible specimen-receiver vessel in which a specimen of fecal matter can be diluted with water or other diluent. The container includes a cover which can be screwed on the specimen-receiver vessel. The cover has an opening passing through it from which samples of diluted fecal matter can be expelled for parasitological analysis by squeezing the sides of the specimen-receiver vessel. A plug is provided for sealing the opening in the cover. A seive is fitted within the cover for straining the solution of fecal matter withdrawn from the container. The orifices of the seive have dimensions which permit the seive to serve in place of gauze or nets of wire or plastic which are used to strain diluted fecal matter in conventional parasitological analysis procedures. A specimen-collector stick is attached to the seive for collecting specimens of fecal matter and introducing them into the specimen-receiver vessel.

The container of the '194 publication would have substantial drawbacks were the container to be used to prepare a specimen of fecal matter for testing for the presence of rotavirus. For example, dispersed in the solution of fecal matter expelled from the container of the '194 publication along with whatever virus particles are present can be bacteria and other non-viral microorganisms, as well as gross particles such as certain intestinal parasites and the eggs of such parasites. Bacteria and non-viral microorganisms are small enough to pass through gauze or net strainers used in parasitological examination procedures, and thus would pass through the seive of the container of the '194 publication. As noted above, the presence of such bacteria or non-viral microorganisms can in certain cases interfere with immunochemical diagnostic test procedures for rotavirus.

Disclosure of the Invention

We have invented a specimen-preparation vial for collecting and preparing a clinical specimen such as a specimen of fecal matter for a diagnostic test which avoids problems of the prior art noted above.

Broadly, the specimen-preparation vial of the invention comprises a specimen-dilution container and a specimen collection/dispenser top for the container.

The specimen-dilution container is shaped to contain a liquid in an interior volume and has an opening dimensioned to permit the specimen to be introduced into the interior volume. A sidewall of the container is yieldable upon manual squeezing.

The specimen-colection/dispenser top includes a cap housing which is connectable to the specimen-dilution container for closing the opening of the container.

The specimen-collection/dispenser top also includes a specimen-collection tool connected to the cap housing. The specimen-collection tool projects from the cap housing so that the tool extends into the interior volume of the specimen-dilution container when the opening of the container is closed by the cap housing.

The specimen-collection/dispenser top further includes a dispenser tip and a dispenser-tip closure. The dispenser tip is connected to the cap housing. The dispenser tip has a channel passing through it. An end of the channel defines a filtrate-discharge orifice of the dispenser tip. The channel through the dispenser tip communicates with the interior volume of the specimen-dilution container when the opening of the container is closed by the cap housing. The dispenser-tip closure is adapted to close the filtrate-discharge orifice of the channel passing through the dispenser tip.

The specimen-collection/dispenser top further includes a microfiltration filter located in the cap housing in a liquid path which extends from the interior volume of the specimen-dilution container to the filtrate-discharge orifice so that liquid which flows from the interior volume of the container to the filtrate discharge orifice passes through the microfiltration filter. The microfiltration filter has pores passing through it of dimensions effective to permit virus particles to pass through the pores and to block the passage of particles significantly larger than virus particles, such as typical bacteria cells. Thus by squeezing the sides of the specimen-dilution container a user can dispense a filtered extract of the specimen in the container, which extract can contain virus particles but which is substantially free of bacteria, other non-viral microorganisms and gross debris.

For use in connection with diagnostic test for rotaviruses, the microfiltration filter of the specimen-preparation vial of the invention preferably has pores of dimensions effective to pass particles having crosswise dimensions of about 53 $\mu$m or less and to block particles having crosswise dimension greater than about 53 $\mu$m. A preferred microfiltration filter is composed of a nonwoven mesh fabric of monofilament nylon. Alternatively, the microfiltration filter may be made of a woven or nonwoven fabric of cotton, rayon or other fiber or filament. If desired, the microfiltration filter may be made of porous ceramic, charcoal, reticulated foam, sintered beads, or other porous material having pores of the appropriate dimensions. If desired, the microfiltration filter may be impregnated with a reagent material such as antibodies or antigens.

Microfiltration filters made of a fabric such as nonwoven mesh of monofilament nylon are prone to shift or wrinkle. Such fabric microfiltration filters are preferably sandwiched between two porous filter-support pads made of a chemically-inert felt material to inhibit shifting and wrinkling.

The specimen-collection tool of the sample-preparation vial of the invention is preferably of a generally flat shape having a depression in one end forming a spoon blade. Such a spoon blade can be used to collect specimens of either solid or runny fecal material. Alternatively, the specimen-collection tool could be a flat paddle, a capillary tube, or an absorbent swab.

The dispenser-tip closure of the specimen-collection/ dispenser top of the specimen-preparation vial of the invention is preferably a break-away-tab plug seal. The cap-housing, dispenser tip and break-away-tab plug seal are preferably molded as a unitary structure. The joint connecting the break-away-tab plug seal to the end of the dispenser tip is made sufficiently thin to permit the plug seal to be broken away readily. Breaking the plug seal away from the dispenser tip opens the filtrate-discharge orifice of the dispenser tip. The break-away-tab plug seal is preferably shaped so that, once broken off from the dispenser tip, it can be inserted in the filtrate-discharge orifice.

Alternatively, the dispenser-top-closure of the specimen-collection/dispenser top may be a snap-on or screw-on cap or an on-off valve mechanism.

The dispenser tip preferably has a generally conical shape to facilitate dispensing the filtrate liquid drop-by-drop. The filtrate-discharge orifice preferably has an inside diameter which is effective to cause the orifice to dispense the filtrate liquid in drops of a volume appropriate for the diagnostic test procedure in which the filtrate is to be used.

The sample-preparation vial of the present invention is inexpensive to manufacture and can be discarded after a single use. The vial can be marketed prefilled with a test reagent such as a buffer solution suitable for diluting a specimen in a particular diagnostic test.

The sample preparation vial of the present invention can be used to advantage in a number of diagnostic tests which require extracts of clinical specimens which are substantially free of bacteria, non-viral microorganisms and gross debris. The sample-preparation vial is particularly suited for use in tests for detecting the presence of rotavirus in specimens of fecal matter, including immunochemical diagnostic tests such as latex-bound-antibody agglutination, immunofluorescence, and enzyme-linked immunosorbent assay techniques, as well as electron microscopy analytical procedures. A preferred sample-preparation vial of the present invention was found to eliminate over 90 percent of the bacteria in a solution of fecal matter. Use of the sample-preparation vial of the invention to prepare a filtered extracts of fecal matter for testing was found to improve significantly the sensitivity and specificity of a latex-bound-antibody agglutination test for the presence of rotavirus in the fecal matter.

Brief Description of the Drawings

Preferred embodiments of the invention are described below with reference to the following drawings.

FIG. 1 is a partially cut away front view of a preferred specimen-preparation vial of the present invention with the specimen collection/dispenser top disconnected from the specimen-dilution container.

FIG. 2 is a partially-exploded cross-sectional view taken along line 2-2 of FIG. 1.

FIG. 3 is a cross-sectional view taken along line 3-3 of FIG. 2.

FIG. 4 is a front view a spoon member of the specimen-preparation vial of FIG. 1.

FIG. 5 is a top view of the spoon member of FIG. 4.

Description of the Preferred Embodiments

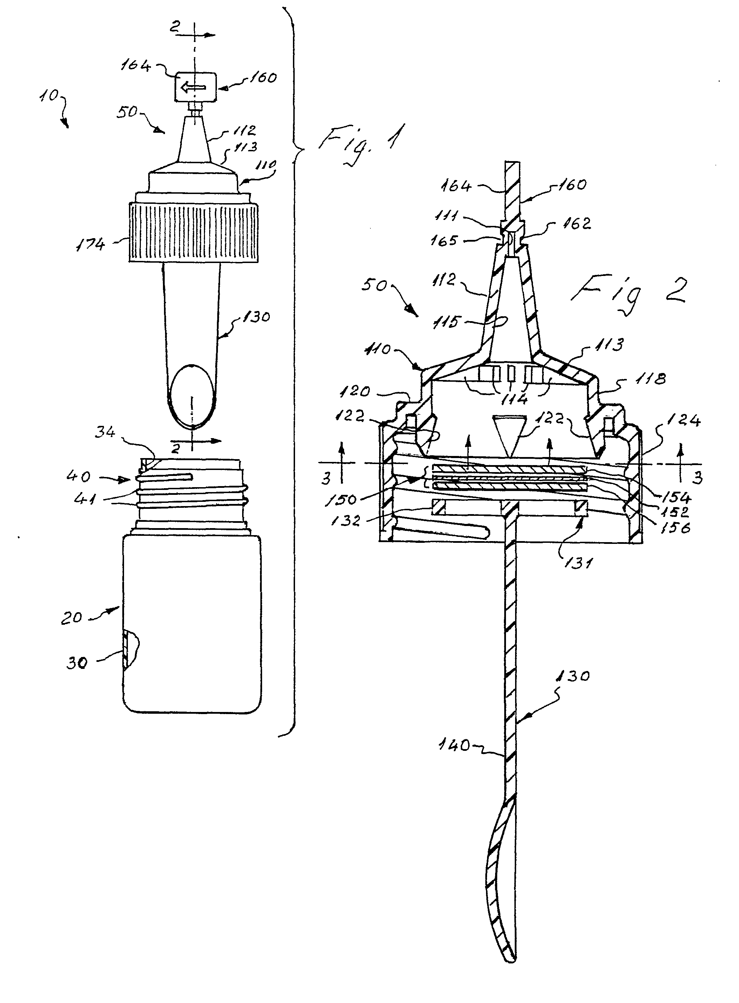

Referring now to FIG. 1, a specimen preparation vial 10 of the present invention comprises a unitary specimen-dilution container 20 and a specimen collection/dispenser top 50.

The specimen-dilution container 20 is a generally cylindrically-shaped vessel molded from polypropylene. The container 20 has a sidewall 30 which is sufficiently thin to yield readily when squeezed by a user, so that, as described below, liquid in the container 20 can be dispensed drop-by-drop by squeezing the sides of the container. The container 20 has an opening 34 which is sufficiently wide in diameter to permit a specimen of fecal matter roughly one ml in volume to be inserted in the container. A neck 40 of the specimen-dilution container 20 is threaded with threads 41 to permit the specimen-collection/dispenser top 50 to be screwed on.

As shown best in FIG. 2, the specimen collection/dispenser top 50 comprises a dispenser cap 110, a specimen-collection spoon member 130, and a filter. assembly 150.

The dispenser cap 110 is a unitary structure molded of polystyrene. The dispenser cap 110 includes a generally conical dispenser tip 112 having a filtrate-dispenser passageway 115 extending through it. A break-away-tab plug seal 160 has a plug shaft 162 and a tab grip 164. The plug shaft 162 is breakably connected to a filtrate discharge end of the dispenser tip 112 by a breakable joint 165. The plug shaft 162 closes off and seals the filtrate-dispenser passageway 115. The break-away-tab plug seal 160 can be detached from the dispenser tip 112 by breaking the plug shaft 162 at the breakable joint 165. Once the plug shaft 162 is broken away, a filtrate-discharge orifice 111 is opened in the dispenser tip 112. Liquid can pass from the filtrate-dispenser passageway 115 through the filtrate-discharge orifice 111 and be dispensed drop-by-drop from the dispenser tip 112. The inside diameter of the filtrate-discharge orifice 111 is selected so that the drops of liquid formed on the dispenser tip 112 have a volume appropriate for the diagnostic procedure in which the drops are to be used. After the break-tab plug seal 160 has been broken away from the dispenser tip, the plug shaft 162 can be inserted in the filtrate discharge orifice 111 to plug the orifice.

The dispenser cap 110 includes a filter-holder cylinder 118 and a cap-sidewall cylinder 124. The diameter of the cap-sidewall cylinder 124 is larger than the diameter of the filter-holder cylinder 118. The two cylinders 118 and 124 are disposed generally coaxially with respect to one another with the filter-holder cylinder 118 extending from a point within the cap-sidewall cylinder 124 to a point beyond the end of the cap-sidewall cylinder 124. An annular container sealing rim 120 connects an end of the cap-sidewall cylinder 124 to a central section of the filter-holder cylinder 118.

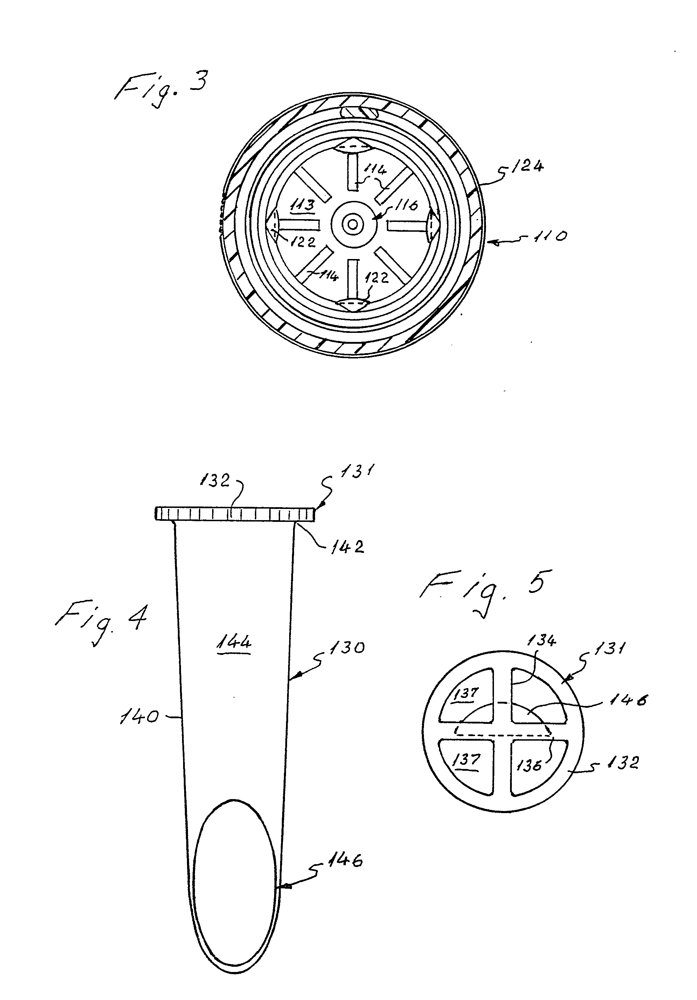

A generally-conical, annular filter-support-rim 113 extends between a base of the dispenser tip 112 and the filter-holder sidewall 118. As may be seen in FIG. 3, eight filter-support ribs 114 extend generally radially from a central opening 116 of the filter-support rim 113 to an outer perimeter of the rim on the side of the rim facing away from the dispenser tip 112. The filter-support ribs 114 are spaced apart from one another azimuthally at approximately equiangular intervals about the central opening 116 in the filter-support rim 113.

Four filter-assembly-retainer tabs 122 project radially inwardly from an inside surface of the filter-holder cylinder 118. The filter-assembly-retainer tabs 122 are spaced apart azimuthally from one another at approximately ninety-degree intervals about the inside surface of the filter-holder cylinder 118 and are spaced apart axially from the filter support ribs 114 by a filter-assembly retainer distance.

The cap-sidewall cylinder 124 of the dispenser cap 110 is threaded on its inside surface to engage the screw threads 41 on the neck 40 of the specimen-dilution container 20. The pitch of the screw threads is sufficiently high to permit the specimen-collection/dispenser top 50 to be screwed on or off the specimen-dilution container 20 in approximately one full rotation. An annular gap between an outer surface of the filter-holder cylinder 118 and an inner surface of the cap-sidewall cylinder 124 defines a container-sealing gap 123. The container-sealing gap 123 is dimensioned to receive and tightly seal a rim around the opening 34 of the specimen-dilution container 20 when the specimen collection/dispenser top 50 is screwed on the specimen dilution container 20.

Turning now to FIGS. 4 and 5, the specimen-collection spoon member 130 comprises a spoon support base 131 and a specimen-collection spoon 140. The spoon support base 131 comprises an annular filter-assembly-retainer rim 132 and first and second cross members 134 and 136 extending across the rim 132. The filter-assembly-retainer rim 132 is dimensioned to fit tightly within the filter-holder cylinder 118 and to engage the filter-assembly-retainer tabs 122.

When the specimen-collection/dispenser top 50 is assembled, the filter assembly 150 is located between the filter-support ribs 114 and the spoon-support base 131. The cross members 134 and 136 of the spoon-support base 131 have approximately the same width as the width of the filter-assembly-retainer rim 132 and divide the area inside the rim into four quadrants with an opening 137 in each quadrant. As a result of this arrangement, the spoon-support base 131 supports the filter assembly 150 while permitting liquid to pass through the openings between the cross members 134 and 136. When the filter-assembly-retainer rim 132 engages the filter-assembly-retainer tabs 122, the spoon-support base 131 holds the filter assembly 150 tightly against filter-support ribs 114, with the retainer rim 132 tending to prevent liquid from flowing around the perimeter of the filter assembly 150 and thereby bypassing the filter.

The specimen-collection spoon 140 of the spoon member 130 is attached to the cross member 136 of the spoon-support base 131. The specimen collection spoon 146 comprises a generally flat spoon shaft 144 which has a depression in a first end to define a spoon blade 146. A second end 142 of the spoon shaft 144 is connected to the cross member 136. The entire spoon member 130 is molded from polypropylene. As shown in FIG. 4, the spoon shaft 144 is tapered slightly in a crosswise dimension. In addition, the spoon shaft 144 is flared at its second end 142 in order to strengthen the joint between the shaft and the cross member 136.

The spoon blade 146 can be used to collect a specimen of fecal matter for diagnosis for rotavirus. With the spoon member 130 held in place in the top 50, the spoon shaft 144 projects axially beyond the end of the cap-sidewall cylinder 124. The cap-sidewall cylinder 124 can be used as a handle for manipulating the spoon member 130, thereby facilitating the collection of a specimen of fecal matter for diagnosis. The shape of the spoon blade 146 permits specimens to be collected from either solid or runny fecal material.

Turning again to FIG. 2, the filter assembly 150 includes a microfiltration filter 152 sandwiched between a first and a second porous filter-support pad 154 and 156. The filter assembly 150 is located adjacent to the filter-support ribs 114 so that the filter is spaced away from the filter-support rim 113. Spaces between the filter support ribs 114 form channels through which filtrate can flow from the filter into the central opening 116 of the filter-support rim 113.

The microfiltration filter 152 is made of a non-woven monofilament nylon filter material which has pores dimensioned particles and to retain larger microorganisms. In particular, the pores of the microfiltration filter 152 tend to pass particles having effective crosswise dimensions of less

than about 53 μm and to block particles having greater effective crosswise dimensions. Consequently, rotavirus particles generally pass through the microfiltration filter 152 while non-virus microorganisms typically found in fecal matter generally do not. The filter support pads 154 and 156 are made of a porous blotting-paper-like felt composed of short-staple, chemically-inert fibers. The filter-support pads tend to prevent the microfiltration filter 152 from wrinkling and shifting. A suitable microfiltration filter material sandwiched between two layers of filter support padding felt is commercially available under the trade designation "CMN 53" from Small Parts Incorporated of Miami, Florida.

The diagnostic-test specimen-preparation vial 10 can be used to advantage to prepare a filtered extract of fecal matter for a latex-bound-antibody agglutination test for the presence of rotavirus in the fecal matter. The use of the specimen-preparation vial will be described below in terms of carrying out such a test procedure, although it will be appreciated that the specimen-preparation vial can be used to similar advantage in other clinical test procedures for virus particles.

Approximately 10 ml of a pH 7.2 buffer solution containing about 0.1 percent sodium azide as a preservative is introduced into the specimen-dilution container 20. A suitable buffer solution is commercially available under the trade name "Rotalex Buffer" from Orion Diagnostica of Espoo, Finland.

The buffer solution may be sealed in the sample-preparation vial 10 for storage for an extended period prior to use by screwing the top 50 on the specimen-dilution container 20 with the break-away-tab plug seal 160 in place. It is anticipated that the specimen-preparation vial of the invention will ordinarily be sold to users prefilled with an appropriate quantity of buffer solution for a single diagnostic test procedure.

To test for the presence of rotavirus in the stools of a patient, the specimen collection/dispenser top 50 is removed from the specimen-dilution container 20. The specimen-collection spoon member 130 is used to collect a specimen of fecal matter roughly one ml in volume from the stools of the patient. The spoon member 130 containing the specimen is then reinserted into the specimen-dilution container 20 and the top 50 is threaded onto the container 20 to seal the container. The specimen preparation vial 10 is then shaken vigorously in order to mix the fecal matter and buffer solution thoroughly and thereby disperse the rotavirus, if present, in the buffer solution.

Next, the break-away-tab plug seal 160 is broken off from the dispenser tip 112. The specimen-preparation vial 10 is then inverted and the sides of the specimen-dilution container 20 are gently squeezed. The squeezing of the container 20 forces the solution of fecal matter against the filter assembly 150 and causes a filtrate of filtered fecal extract solution to pass though the filter assembly and flow down the filtrate-dispenser passageway 115 to the filtrate-discharge orifice 111 in the dispenser tip 112. Filtered fecal extract solution is then dispensed drop-by-drop from the filtrate discharge orifice 111 of the dispenser tip 112 by squeezing the sides of the specimen-dilution container 20. The filtered fecal-extract solution is substantially free of bacteria and gross debris, but contains rotavirus particles if the solution of fecal matter in the specimen-dilution container 20 contains rotavirus particles.

The first eight drops of filtered fecal extract solution are discarded. The next two drops are placed respectively on two separate areas of a black, nonabsorbent card. One drop of a rotavirus-sensitive agglutination test reagent is applied to one of the drops of filtered fecal-extract solution on the card and one drop of a negative control reagent is applied to the other drop of filtered fecal-extract solution. The test reagent is a suspension of latex particles to which rabbit anti-rotavirus antibodies are bound in a medium which contains about 0.1 percent sodium azide as a preservative. A suitable test reagent is commercially available under the trade name "Rotalex Test Latex Reagent" from Orion Diagnostica identified above. The negative control reagent is a suspension of albumin bound to latex particles in a medium which contains about 0.1 percent sodium azide as a preservative. A suitable negative control reagent is commercially available under the trade name "Rotalex Non-Reactive Latex Reagent" from Orion Diagnostica. Each of the two pairs of drops is then stirred gently using a separate clean mixing paddle for each pair to avoid cross mixing. The test card is then tilted and rotated for about two minutes to cause the reagents to move in a circular pattern. The presence of agglutination of latex particles in the sample containing the test reagent and the absence of agglutination in the sample containing the negative control indicates that the fecal material contained rotavirus.

Following the test, the break-tab plug seal 160 can be inserted in the filtrate-discharge orifice 111 of the dispenser top 112 to seal the specimen preparation vial 10. The vial thus sealed can then be discarded with a minimum risk of spillage or infection of personnel administering the test or contamination of the surrounding environment.

It is not intended to limit the present invention to the specific embodiment described above. For example, the cap housing can be connected to the specimen-dilution container by a snap-on action.  It is recognized that this and other changes may be made in the specimen-preparation vial specifically described herein without departing from the scope and teaching of the instant invention, and it is intended to encompass all other embodiments, alternatives and modifications consistent with the invention.

Claims

1. A specimen-preparation vial for collecting and preparing a specimen for a diagnostic test, comprising:

(a) a sample-dilution container shaped to contain a liquid in an interior volume, the container having an opening dimensioned to receive the specimen, a sidewall of the container being yieldable upon manual squeezing; and

(b) a specimen-collection/dispenser top including;

(b.1) a cap housing connectable to the specimen-dilution container for closing the opening of the container;

(b.2) a specimen-collection tool connected to the cap housing, the specimen-collection tool projecting from the cap housing so that the specimen-collection tool extends into the interior volume of the specimen-dilution container when the cap housing closes the opening of the container;

(b.3) a dispenser tip connected to the cap housing, the dispenser tip having a channel passing through it which communicates with the interior volume of the sample-dilution container when the cap housing closes the opening of the container, an end of the channel defining a filtrate-discharge orifice;

(b.4) dispenser-tip closure means for closing the filtrate-discharge orifice of the channel passing through the dispenser tip; and

(b.5) a microfiltration filter located in the cap housing in a liquid path extending from the interior volume of the sample-dilution container to the filtrate discharge orifice of the channel passing through the dispenser tip so that liquid flowing from the interior volume of the container to the filtrate-discharge orifice passes through the microfiltration filter, the microfiltration filter having pores passing through it of dimensions effective to permit virus particles to pass through the pores and to block the passage of particles significantly larger than virus particles.

2. The specimen preparation vial of claim 1 in which the microfiltration filter comprises a nonwoven fabric mesh of a monofilament nylon.

3. The specimen-preparation vial of claim 1 or 2 in which the pores of the microfiltration filter are dimensioned so that particles having effective crosswise dimensions of about 53 $\mu$m or less tend to pass through the pores and particles having effective crosswise dimensions of greater than about 53 $\mu$m tend to be blocked.

4. The specimen-preparation vial according to any of claims 1 to 3 in which the microfiltration filter is sandwiched between two porous filter-support pads.

5. The specimen-preparation vial of claim 4 in which the filter-support pads are comprised of a blotting-paper-like felt material.

6. The specimen-preparation vial according to any of claims 1 to 5 in which the dispenser-tip closure means is a break-away tab plug seal connected to an end of the dispenser tip by a breakable joint to close off and seal the channel passing through the dispenser tip, the break-away-tab plug seal having a plug shaft adapted to be inserted in the filtrate-discharge orifice at the end of the channel passing through the dispenser tip to plug the orifice after the break-away-tab plug seal has been broken away from the dispenser tip.

7. The specimen-preparation vial of claim 6 in which the dispenser tip is generally conical in shape.

8. The specimen-preparation vial according to any of claims 1 to 7 in which the specimen-collection tool is detachably connected to the cap housing.

9.   The specimen-preparation vial of claim 8 in which the specimen-collection tool has a tool-support base which cooperates with the cap housing to retain the microfiltration filter in the cap housing.

10. The specimen-preparation vial of claim 8 or 9 in which the specimen-collection tool is shaped to form a spoon.

½

Fig. 1

Fig 2

Fig. 3

Fig. 4

Fig. 5